# EUROPEAN PATENT APPLICATION

(11) **EP 1 199 030 A2**
(43) Date of publication of application: **24.04.2002**
(21) Application number: 01308853.9
(22) Date of filing: 18.10.2001
(51) Int. Cl.: A61B 5/0432

(54) **Monitoring apparatus for human body function**

(30) Priority: 19.10.2000 US 693283; 20.10.2000 GB 0025778
(71) Applicant: Reynolds Medical Limited, Hertford SG13 7HW (GB)
(72) Inventor: Carter, Hugh, Edinburgh EH7 4HN (GB)
(74) Representative: Foster, Mark Charles

(57) **Abstract**

ECG recording apparatus 1 includes a port 2 for connection to ECG electrodes and a microphone 3 for detecting vocal signals. Processing electronics store a recording of the vocal signals and the physiological data on the storage medium 4.

## Description

This invention relates to methods and apparatus for recording physiological information, and more particularly to ECG recording apparatus which stores a recording of a vocal signal and physiological information.

Body monitoring apparatus generally includes physiological sensors, a monitoring device and cables connecting the sensors to the monitoring device. The sensors are attached to a patient's body to detect physiological signals and may be, for example, electrocardiogram (ECG) electrodes, electroencephalogram (EEG) electrodes or blood oxygen sensors. For example, in ECG monitoring apparatus an electrocardiogram signal is measured as the difference in potential between a set of electrodes placed externally on the body of the patient. This allows the cardiac activity to be measured.

In ambulatory monitoring the complete electronic apparatus is miniaturised, battery operated, and adapted for wearing on the patient's body. WO/94/26164 describes one known ambulatory monitoring apparatus, and is incorporated herein by reference.

US 5314389 discloses an ECG monitor that gives the user an audible indication of pulse rate. US 5474090, US 4535783, US 4183354, US 4087840 and US 3651280 disclose monitoring systems that record physiological information and voice information.

It is advantageous to be able to quickly input and store data which relates to the recording of the physiological information. For example, to identify the recording, the patient, medication, symptoms, and the clinician.

The invention is defined in the independent claims.

The invention enables quick and easy labelling a recording of physiological information. The labelling used provides the name or other identification means of the human or animal subject of the recording. It may in addition supply information such as the identity of the clinician/hospital performing the recording, the type of data analysis that should be performed on the physiological data (such as "test for heart-pacemaker failure") and additional data such as a patient's medication or symptoms.

According to one aspect of the present invention, there is provided a method of gathering physiological information from a human or animal subject, the method including: declaring the name or other identification means of the human or animal subject verbally, detecting and storing said verbal signal using acoustic and electrical means, detecting and storing physiological information from the subject and associating the verbal recording with the physiological information recording. The vocal signal acts as the labelling method for the stored physiological information. The vocal recording labelling may also include elements for identifying the clinician and/or hospital performing the recording, and the type of analysis that should be applied to the recorded physiological data.

According to a another aspect of the present invention, there is provided a method of monitoring physiological information, the method including:
a) gathering physiological information and an associated verbal recording as described in the first aspect;
b) transmitting both the physiological information and the vocal signal to a remote location (typically referred to as a 'receiving station');
c) said receiving station using the vocal signal to identify the subject of the recording;
d) said receiving station using the vocal signal to identify the clinician conducting the physiological recording (for the purposes of determining where and how the response from the receiving station should be sent, billing information etc.);
e) said receiving station using the vocal signal to label the recording, either directly (an acoustic label) or via a transcription process; and
f) said receiving station using the vocal signal to identify what type of analysis should be applied to the physiological information.

According to a further aspect of the present invention, there is provided body-monitoring apparatus including: acoustic and electronic means for receiving and recording a vocal signal as described in the first-mentioned aspect, and processing electronics for receiving and recording physiological information from physiological sensors connected to the apparatus.

The apparatus may include a loudspeaker such that the clinician may replay the vocal recording to verify the vocal labelling used and re-record it if necessary.

The processing electronics may be adapted for supplying and/or restricting power to the acoustic sensor(s) in response to user commands received through an interface. This allows the power supply of the apparatus to be conserved.

For a better understanding of the present invention, specific embodiments will now be described by way of example, with reference to the accompanying drawings, in which:
Fig. 1 shows body monitoring apparatus according to the invention connected to ECG electrodes; and
Fig. 2 shows a schematic circuit design of body monitoring apparatus according to the invention.

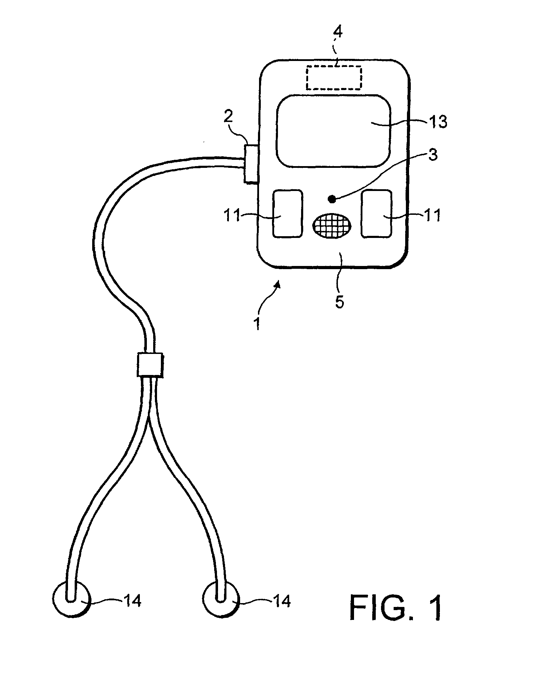

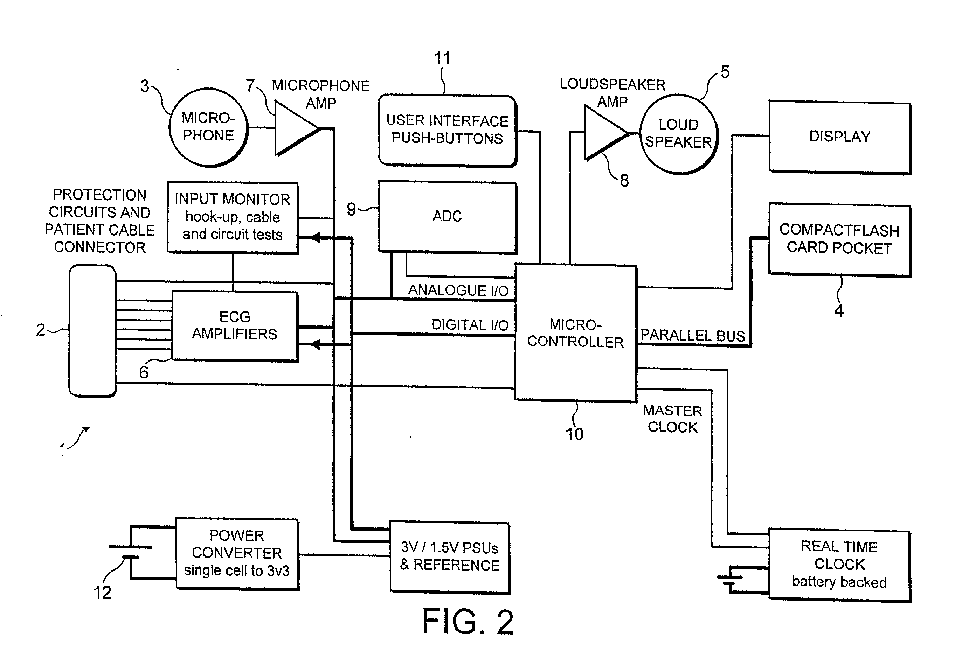

Referring to the embodiment in Figs. 1 and 2, portable ECG recording apparatus 1 includes a port 2 for connection to ECG electrodes 14, a microphone 3 for detecting a sound signal, a solid state memory card 4 and a loudspeaker 5. The apparatus 1 also includes processing electronics which include amplifiers 6, 7, 8, an analogue to digital converter (ADC) 9 and a microprocessor 10.

ECG electrodes 14 are attached to the patient's body to detect physiological information, and are connected via port 2 to ECG recording apparatus 1.

Prior to recording physiological information, the clinician activates the microphone 3 using the user interface controls 11, which causes the processing electronics to provide power to the microphone 3 from a battery 12. Vocal signals from the clinician are detected by the microphone 3, and the analogue signals are fed through the microphone amplifier 7. The amplified signals are digitised using the ADC 9 and stored on the memory card 4. The vocal recording may be compressed in order to save storage space on the memory card. An LCD display 13 displays user options and system status.

The clinician is able to replay the vocal recording if desired, and if it is not suitable it may be re-recorded. In order to replay the vocal recording, the digitised signal is reconstructed including being passed through the loudspeaker amplifier 8, and is acoustically output through the loudspeaker 5. The microprocessor 10 deactivates the loudspeakers when not in use to save power.

The clinician is therefore able to make a vocal recording to store any information which is relevant to the subsequent physiological data recording. This method of data input provides a very simple and practical method for the clinician without recourse to a keyboard or a large number of button operations. Once the clinician is satisfied with the vocal recording, the microphone 3 may be deactivated in order to lower the power requirements.

The microphone 3 device characteristics may vary depending on the requirements, for example with the acoustic input level set so the device can record speech clearly at a distance of 10-30cm from the clinician's mouth. In the embodiment, the microphone response is +/- 3dB over the range 600 Hz to 2 kHz. The speech signal is sampled and recorded at 8 kHz.

The loudspeaker may have any suitable characteristics required, for example the acoustic output capability of 70 dBA, 10 cm from the output port. The loudspeaker response in the embodiment is +/- 10 dB over the range 600 Hz to 3 kHz.

Physiological signals detected by the sensors are fed through ECG amplifiers 6 and digitised using the same analogue to digital converter 9 as is used to digitise the vocal signal. The digitised physiological data is stored on the memory card 4, which is the same storage medium as the vocal recording. Thus, the memory card 4 contains recordings of both the vocal signal and the physiological data and these will be kept together, even if the data is transferred to a remote site, so that the recordings do not become accidentally separated.

Since both the vocal signal and ambulatory recording are stored on the same storage medium, this provides a unique identification scheme for the recording. If the data is sent to a remote site, it is possible to identify both the recording and its source using the information held in the vocal recording. Physiological symptoms and medication details may also be relayed if these have been recorded. In the case of trans-telephonic event recording, the message can be chosen by the clinician to satisfy any identification requirements of the receiving station. The message allows the receiving station to automatically identify the patient and therefore the system can react to the physiological data that will follow in the appropriate manner. This avoids relying on the patient to perform this function. This advantageously minimises patient interaction, which may be important if the patient is in distress, avoiding the time required to teach the patient the appropriate responses, and avoiding any error caused by patient confusion or misinterpretation of patient's speech.

In the embodiment, the loudspeaker can also replay sound recordings previously stored in the apparatus. These recordings may be supplied, for example, by the device manufacturer, uploaded from a personal computer or recorded directly on the recorder. The loudspeaker can output prerecorded vocal signals, or sound signals (beeps) indicating successful/failed operations, button pushes or self-diagnostic error messages. For example, vocal messages from the recorder may be triggered on the basis of the recorder status. These include requesting the clinician to check recording inputs before starting recording, warning that a sensor is not connected, or prompting a change in battery.

In the embodiment, the recorder incorporates an analysis system where automatically detected physiological conditions will trigger the replay of an appropriate vocal recording prompting the patient to certain actions. These may be, for example, downloading of data or contacting a doctor.

Although in the embodiment, the storage medium is a solid state memory card, any other suitable storage medium may be used instead.

## Claims

1. A method of monitoring physiological information, the method including: detecting and recording physiological information from a human or animal subject using at least one physiological sensor; detecting and recording a vocal signal using an acoustic sensor, said vocal signal including a component suitable for the identification the human or animal subject of the recording.

2. Body monitoring apparatus including: processing electronics for receiving and recording physiological information from a human or animal subject using at least one physiological sensor; processing electronics for receiving and recording a vocal signal from a sound sensor, said vocal signal including a component suitable for the identification the human or animal subject of the recording.

3. A method or apparatus according to claim 1 or 2, wherein, the vocal recording contains a component suitable for the identification of the physician, clinician, hospital, clinic, company or the like for whom the monitoring is performed.

4. A method or apparatus according to claim 1, 2 or 3, wherein the vocal recording is stored on the same recording medium as the physiological recording.

5. A method or apparatus according to claim 1, 2, 3 or 4, wherein the vocal recording is the primary means of identifying the human or animal subject of the recording.

6. A method or apparatus according to any one of claims 1 to 5, wherein the vocal recording is the primary means of identifying the physician, clinician, hospital, clinic, company or the like for whom the monitoring is performed.

7. A method or apparatus according to any one of claims 1 to 6, where the vocal recording is made before or after, not concurrent with, the recording of the physiological information.

8. A method according to any one of claims 1 and 3 to 7, including the step of replaying the vocal recording once made, and overwriting the vocal recording with a new vocal recording if the initial vocal recording is not considered to be adequate.

9. A method according to any one of claims 1 and 3 to 8, including a step wherein power is supplied to the acoustic sensor only when a vocal recording is to be made.

10. A method according to any one of claims 1 and 3 to 9, including a step wherein power is restricted from the acoustic sensor when a vocal recording is not being made.

11. A method according to any one of claims 1 and 3 to 10, including the additional step of transmitting both the recorded physiological information and the recorded vocal signal to a remote location in such a manner that the vocal recording is directly associated with the recorded physiological information.

12. A method according to any one of claims 1 and 3 to 11, wherein the vocal recording and the transmitted physiological recording are stored as associated data and the vocal recording is used directly as an acoustic label for the physiological recording.

13. A method according to claim any one of claims 1 and 3 to 11, wherein the vocal recording is transcribed in whole or in part, and said transcription is used to label the transmitted physiological recording, either by associated storage of the transcribed data with the physiological recording or as appended, prefixed or otherwise encoded data within the stored physiological recording.

14. A method of monitoring physiological information according to any one of claims 1 and 3 to 11, wherein an additional component of the vocal recording is used to define the method of data analysis that should be applied to the recorded physiological information.

15. Body monitoring apparatus according to any one of claims 2 to 7, including a loudspeaker and processing electronics such that the vocal recording, or a previously stored sound signal, may be replayed through the loudspeaker.

16. Body monitoring apparatus according to any one of claims 2 to 7 or 15, the processing electronics adapted for supplying power to activate the acoustic sensor only when a vocal recording is to be made.

17. Body monitoring apparatus according to any one of claims 2 to 7, 15 or 16, the processing electronics adapted for restricting power to deactivate the acoustic sensor when a vocal recording is not being made.

18. Body monitoring apparatus according to any one of claims 2 to 7, 15 or 16, comprising means to transmit both the recorded physiological information and the recorded vocal signal to a remote location in such a manner that these recordings are directly associated with each other.

19. A method of monitoring patient physiological information, the method including:
storing patient physiological data from physiological sensors;
storing a sound signal capable of identifying the nature of patient physiological data to a remote receiving station;
transmitting the stored sound signal and patient physiological data to the remote receiving station; and
at said remote receiving station interpreting said sound signal to identify the nature of said patient physiological data so that the data can be processed in a manner selected according to the nature of the patient's physiological data.
